# EUROPEAN PATENT APPLICATION

(11) **EP 2 151 220 A1**
(43) Date of publication of application: **10.02.2010**
(21) Application number: 08764865.5
(22) Date of filing: 29.05.2008
(51) Int. Cl.: A61F 13/15, A61F 13/515

(54) **ABSORPTIVE ARTICLE**

(30) Priority: 30.05.2007 JP 2007143547
(71) Applicant: Unicharm Corporation, Ehime 799-0111 (JP)
(72) Inventor: KURODA, Kenichiro, Kanonji-shi Kagawa 769-1602 (JP); NODA, Yuki, Kanonji-shi Kagawa 769-1602 (JP); NISHIKAWA, Kumiko, Kanonji-shi Kagawa 769-1602 (JP)
(74) Representative: Sperling, Rüdiger
(86) International application number: PCT/JP2008/059896
(87) International publication number: WO 2008/149771

(57) **Abstract**

Provided is an absorptive article, which can be prevented from having a liquid leakage or from being broken at the side edges along its longitudinal direction and which can keep softness at its seal portion thereby to reduce an uncomfortable feeling. In a seal portion (50) formed along the outer peripheral edge of the absorptive article (1), a plurality of contact-bonded portions (51) are formed at a spacing from each other. Of these plural contact-bonded portions (51), a first contact-bonded portion (53) and a second contact-bonded portion (54) adjacent to the first contact-bonded portion (53) in the width direction (WD) of the absorptive article (1) are arranged to have a mutually overlapping region in the width direction (WD).

## Description

### TECHNICAL FIELD

The present invention relates to an absorbent article.

### BACKGROUND ART

Absorbent articles such as a sanitary napkin or a sheet for absorbing vaginal discharge have been widely known as absorbents for absorbing liquids such as the discharged matter discharged from the vaginal opening of females. Absorbent articles include liquid retentive absorbent layers, liquid-permeable top sheets and liquid-impermeable back sheets, in which the perimeter thereof is sealed with the heat embossment processing or the like. The sealed portion thus sealed is required to maintain the strength so as to prevent itself from breaking while in use, and also required not to make the wearer uncomfortable in use.

Conventionally, in order to seal the perimeter of the absorbent article, a method has been widely known in which the sealed portion is formed in a planar shape by heat embossing process or the like. However, the sealed portion formed in this way is hardened due to the compression in corresponding regions by the embossing process, as a result of which there are cases where the wearer feels uncomfortable while using the absorbent article. Furthermore, the hardening of the corresponding regions makes it hard for the absorbent article to deform along the curve of the wearer's body, thereby causing a gap between the body and the absorbent article, also resulting in leakage.

Accordingly, an invention has been disclosed, in which instead of the embossing process in a planar shape, a plurality of substantially square concave portions are formed by the embossing process, as a result of which the number of folding-starting points is increased, which are formed in convex portions adjacent each other between concave portions in the sealed portions, thereby making it easy for the absorbent article to deform along the curve of the wearer's body for example, Japanese Unexamined Patent Application Publication No. 2001-129019. Moreover, the concave portions are arranged to the adjacent concave portions in serial, such that an area per concave portion is larger in portions with greater basis weight or thickness, and an area per concave portion is smaller in the other portions.

### DISCLOSURE OF THE INVENTION

### Problems to be Solved by the Invention

In Japanese Unexamined Patent Application Publication No. 2001-129019, the concave portions with greater basis weight are formed to be relatively large. As a result, the area is increased due to the heat, thereby the wearer may feel uncomfortable while using the absorbent article. Moreover, as the area per concave portion becomes large, the distance between folding-starting points that are formed in convex portions is widened. Since this decreases the flexibility in the perimeter, thereby making it harder for the absorbent article to deform along the curve of the wearer's body, it is apprehended that a gap is caused, resulting in leakage.

Moreover, in cases where the concave portions are formed along the longitudinal direction in the side edges of the absorbent article, regions, in which the concave portions are not formed and which became relatively convex portions, are formed in a straight line extending in the width direction. As a result, in cases where liquids such as the discharged matter flow in, the liquids are led along the regions that became the convex portions, thereby leaking the liquids.

Thus, an object of the present invention is to provide an absorbent article, which makes it possible to prevent the leakage of liquids and the folding of the absorbent article in the side edges along the longitudinal direction of the absorbent article. Moreover, another object of the present invention is to provide an absorbent article, which makes it possible to maintain the flexibility in the sealed portion and to reduce the uncomfortability.

### Means for Solving the Problems

In a first aspect of the present invention, an elongated absorbent article is provided, which includes a top sheet portion having a seat, at least a part of which is liquid-permeable; a back sheet portion having a liquid-impermeable seat; and a liquid retentive absorbent core which is arranged between the top sheet portion and the back sheet portion, a joined region formed in outer perimeter of the absorbent article, which joins the back sheet portion and the top sheet portion; and a plurality of pressure-bonding parts formed in a separated way in all or part of the joined region, and in which each of the plurality of pressure-bonding parts formed in the outer perimeter in a width direction of the absorbent article has a region overlapping with an pressure-bonding part adjacent to each other in the width direction.

In a second aspect of the absorbent article as described in the first aspect of the present invention, the absorbent article is provided, in which the plurality of pressure-bonding parts has a plurality of first pressure-bonding parts formed along the outer perimeter and a plurality of second pressure-bonding parts formed to be substantially parallel to the first pressure-bonding parts, and in which the second pressure-bonding parts are formed to intersect a straight line extending in a width direction from one end of a longitudinal direction of the first pressure-bonding parts, and not to intersect a straight line extending in the width direction from the other end of the longitudinal direction.

In a third aspect of the absorbent article as described in the second aspect of the present invention, the absorbent article is provided, in which, in the longitudinal direction, the length of the first pressure-bonding parts are different from the length of the second pressure-bonding parts.

In a fourth aspect of the absorbent article as described in any one of the second or third aspect of the present invention, the absorbent article is provided, which further includes third pressure-bonding parts, each of which is arranged between a predetermined first pressure-bonding part in the plurality of first pressure-bonding parts and the other first pressure-bonding part adjacent to the predetermined first pressure-bonding part, and of which the length is different in the longitudinal direction from the length of the first pressure-bonding parts.

In a fifth aspect of the absorbent article as described in any one of the first to fourth aspects of the present invention, the absorbent article is provided, in which all or part of the plurality of pressure-bonding parts has a shape elongated in the longitudinal direction of the absorbent article.

In a sixth aspect of the absorbent article as described in any one of the first to fifth aspects of the present invention, the absorbent article is provided, in which the plurality of pressure-bonding parts are formed in the entire area of the outer perimeter, and have regions overlapping with adjacent pressure-bonding parts in the width direction with respect to any position in the longitudinal direction of the absorbent article.

### Effects of the Invention

According to the invention, it is possible to provide an absorbent article which makes it possible to prevent the leakage of liquids and the folding of the absorbent article in the side edges along the longitudinal direction of the absorbent article. Moreover, according to the present invention, it is possible to provide an absorbent article, which makes it possible to maintain the flexibility in the sealed portion and to reduce the uncomfortability.

### BRIEF DESCRIPTION OF THE DRAWINGS

Fig.1 is a plan view for showing a first embodiment of the absorbent article of the present invention;
Fig. 2 is a cross-sectional view of Fig. 1 taken along the line Z-Z;
Fig. 3 is a diagram for showing part of the method of manufacturing the absorbent article of the present invention;
Fig.4 is a plan view for showing an original absorbent article after forming the sealed portion in the manufacturing method of Fig. 3;
Fig.5 is a plan view for showing a second embodiment of the absorbent article of the present invention;
Fig.6A is a plan view for showing a third embodiment of the absorbent article according to the present invention; and
Fig.6B is a enlarged plan view for showing a pressure-bonding part of the third embodiment of the absorbent article.

### PREFERRED MODE FOR CARRYING OUT THE INVENTION

### 1. First Embodiment

### 1-1. Overall Configuration of Absorbent Article

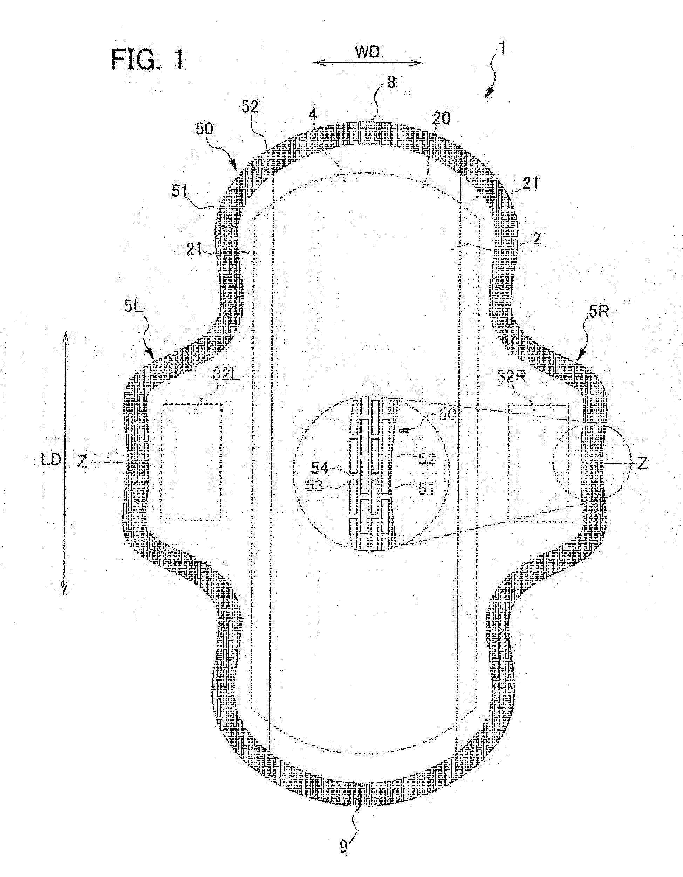

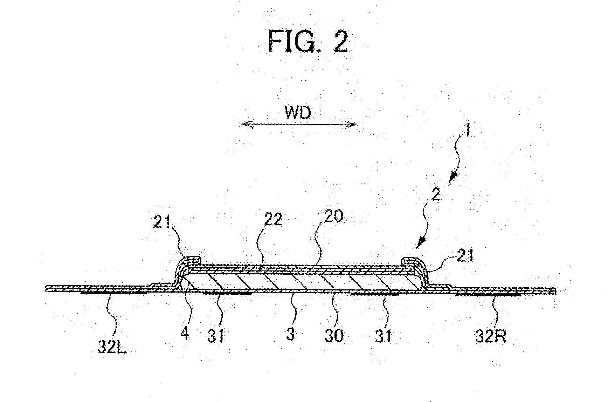

As shown in Fig. 1 and Fig. 2, an absorbent article 1 according to the present invention is formed in an elongated-shape. The absorbent article 1 at least includes a top sheet portion 2 which is at least partly liquid-permeable, a back sheet 3 portion (referring to Fig.2) which serves as a leak proof layer, and an absorbent core 4 which absorbs and holds liquids such as the discharged matter.

The top sheet portion 2 and a back sheet 30 to be described later that constitutes the back sheet portion 3 are joined along the outer perimeter by the heat embossing process, thereby forming sealed portion 50 that is a joined region.

As shown in Fig. 1 and Fig. 2, the top sheet portion 2 is constituted with a top sheet 20 which is arranged in the middle of the width direction WD of the absorbent article 1, side sheets 21 which are arranged along the longitudinal direction LD on both sides of the width direction WD of the top sheet 20, and a second sheet 22 which is arranged between the top sheet 20 and the absorbent core 4.

Top sheet 20 is a liquid-permeable seat, is arranged in such a way to cover the absorbent core 4 and the second seat 22, and is a seat which, in the absorbent article 1, contacts with the wearer's body. It is possible to use the top sheet 20 without a particular limitation as long as it is a seat with a liquid-permeable structure. Moreover, since the top sheet 20 is a member which is in contact with the wearer's body, it is preferable to have the feel that does not cause uncomfortability for the wearer.

Specifically, as the top sheet 20, it is possible to use a non-woven fabric in which a plurality of groove portions are formed with substantially equal intervals on one side of the non-woven fabric, and a plurality of convex portions are formed in each of the plurality of groove portions. It is preferable that a plurality of openings be formed with predetermined intervals in each of the plurality of groove portions. It is possible to form such a non-woven fabric which has a plurality of groove portions and a plurality of convex portions, and in which a plurality of openings are formed in each of the groove portions, by performing the following. For example, a fibrous web, which is a fiber aggregate, is supported from a bottom side by a netted supporting member having breathability, followed by blowing a gas from a top side, thereby moving fibers constituting the fibrous web. This fiber aggregate is like a sheet, and has a degree of freedom to which the fibers can move inside the fiber aggregate. Fiber orientation, fiber sparsity and density, or basis weight of the fibers is adjusted appropriately. Since the groove portions are formed on the top sheet 20, and the openings are provided in the groove portions, it is possible to obtain the absorbent article 1 with superior liquid permeability. Moreover, in cases where such a non-woven fabric is used as the top sheet 20, it is preferable to be used in such a way that the direction in which the groove portions extend coincide with the longitudinal direction LD of the absorbent article 1. By causing the direction in which the groove portions extend to coincide with the longitudinal direction LD of the absorbent article 1, it is possible to prevent the discharged bodily fluids from widely diffusing to the width direction WD of the absorbent article 1.

It should be noted that, in cases where fibers are arranged and rearranged by blowing an air stream, a fibrous web, which is formed by the card method that uses comparatively long fibers, is suitable. In order that after groove portions (concave and convex portions) are formed by a plurality of air flows, the fibers are made into a nonwoven fabric with its shape retained, it is preferable to employ throw air method where thermoplastic fibers are subjected to hot melting by oven processing (heat treatment). Fibers suitable for this manufacture method are preferably those having the core-in-sheath structure or side-by-side structure in order to heat-seal intersecting points of the fibers or are more preferably those having the core-in-sheath structure in which sheaths can be easily heat-sealed in a secure manner. Particularly, a sheath-core composite fiber composed of polyethylene terephthalate and polyethylene, or a sheath-core composite fiber composed of polypropylene and polyethylene can be used suitably. These fibers can be used singly or in combination of two or more types. The fiber length is preferably 20 to 100 mm, especially 35 to 65 mm.

The top sheet 20 causes liquids such as the discharged matter to permeate into the side of the absorbent core 4. The top sheet 20 is joined to an absorbent core 4 to be described later by the embossing process.

The side seats 21 and 21 are arranged along the longitudinal direction LD of the absorbent article 1, on both sides of the width direction WD of the top sheet 20. Parts of the side seats 21 and 21 in the longitudinal direction LD of the absorbent article 1 protrude toward the width direction WD, and respectively form wings 5L and 5R together with the back sheet 30 to be described later.

The side seats 21 and 21 are joined to the top sheet 20 with a hot melt adhesive and the embossed parts (not shown) formed at substantially equal distance along the longitudinal direction LD, on both sides of the width direction WD of the top sheet 20. In detail, as shown in Fig. 2, the side sheets 21 and 21 are folded back at the portions where the side sheets overlap the top sheet 20 and the absorbent core 4, such that the inward regions located in the side sheets inward to the width direction WD of the absorbent article 1 serve as the folding edges. The side seats 21 and 21 are joined to the top sheet 20, of which the outer edges of the absorbent article 1 in the width direction WD extend toward the outside of the absorbent core 4.

It is possible to use the member constituting the side seats 21 without a particular limitation as long as it is a liquid-impermeable seat. As the side seats 21, it is possible to use, for example, water-repellent through-air non-woven fabric and polypropylene spunbonded nonwoven fabric (PPSB).

The second seat 22 is arranged between the top sheet 20 and the absorbent core 4. The second seat 22 is arranged in a state where the liquid-permeable seat is folded in two. As the second seat 22, it is possible to use a conventionally well-known seat as appropriate, for example, such as a seat member which is similar to the top sheet 20. Moreover, it is preferable that the second seat 22 be formed such that the density is higher than that of the top sheet 20. The density of the second seat 22 is higher than that of the top sheet 20, thereby making it possible to increase the liquid-transport property from the top sheet 20.

It is possible to arrange the second seat 22 in such a way to cover the entire surface of the absorbent core 4, and it is not limited thereto as long as the size thereof does not exceed the size of the absorbent article 1. In the present embodiment, the second seat 22 covers the entire surface of the absorbent core 4 in a state where the second seat 22 is folded in two, and the outer perimeter of the second seat 22 substantially corresponds to the outer perimeter of the absorbent core 4. Moreover, the length of the second seat 22 in the longitudinal direction LD is substantially the same as the length of the top sheet 20 in the longitudinal direction LD.

The length of the top sheet 20 and the side seats 21 in the longitudinal direction LD is formed to be greater than the length of the absorbent core 4 in the longitudinal direction LD. Moreover, the length of the top sheet 20 in width direction WD is formed to be the same as, or greater than, the length of the absorbent core 4 in width direction WD.

The back sheet portion 3 constitutes the leakproof layer in the absorbent article 1. The back sheet portion 3 includes a liquid-impermeable back sheet 30, dislocation-preventing portions 31 that fix the absorbent article 1, and wing-side dislocation-preventing portions 32L and 32R.

The back sheet 30 is formed, for example, of a liquid-impermeable seat such as a plastic film, and controls so that liquids such as the discharged matter held in the absorbent core 4 do not adhere to the underwear. As a member to be used for the back sheet 30, specifically, it is possible to use a polyethylene film with a basis weight of 27 g/m²

The dislocation-preventing portions 31 are arranged, in the back sheet 30, at the side opposite to the side where the absorbent core 4 is arranged, and fix the absorbent article 1 by abutting the underwear. Moreover, as for the wing-side dislocation-preventing portions 32L and 32R, after the absorbent article 1 is arranged at the crotch area of the underwear, the wings 5L and 5R are folded back to the side of the underwear, thereby fixing the absorbent article 1 to the underwear. As a member to be used as dislocation-preventing portions 31 and wing-side dislocation-preventing portions 32L and 32R, it is possible to use hot-melt adhesive and male members of a hook-and-loop fastener. Specifically, coater coating is performed using a rubber-based hot-melt adhesive, in which the basis weight is 25 g/m², the coating width is 5 mm, and the coating distance is 5 mm.

Absorbent core 4 is arranged by the width direction WD center of absorbent article 1, liquid such as discharged matter excreted by wearer's body is taken in, and it is held. The size of the absorbent core 4 is formed so that the lengths in the longitudinal direction LD and the width direction WD are smaller than those of the top sheet portion 2 to be described later. Specifically, it is possible to use 400 g/m² of softwood kraft pulp (of which SAP is 10%) wrapped with a tissue. As a tissue for covering the absorbent core 4, it is possible to use, for example, a tissue with a basis weight of approximately 18 g/m². In the first embodiment, a plurality of pores are formed in the tissue, the pores extending in the longitudinal direction LD on the skin contacting side of the absorbent article 1, and the pores penetrating the absorbent article 1 in the thickness direction.

The wings 5L and 5R are formed to protrude outward to the width direction WD, on the respective sides of the absorbent article 1 in the width direction WD. The wings 5L and 5R are formed of the side seats 21 and the back sheet 30, on both sides of the top sheet 20 in the width direction WD.

The sealed portion 50 is formed along the outer perimeter of the absorbent article 1. It is preferable that the absorbent article 1, including the wings 5L and 5R, as a whole be formed with a curved outer periphery.

The length of the back sheet 30 in the longitudinal direction LD is formed to be greater than the length of the absorbent core 4 in the longitudinal direction LD. Moreover, the length of the top sheet 20 and the side seats 21 constituting the top sheet portion 2, in the longitudinal direction LD, is formed to be greater than the length of the absorbent core 4 in the longitudinal direction LD. In other words, the absorbent core 4 is not arranged in a front edge 8 and a rear edge 9 of the absorbent article 1 in a state where the top sheet portion 2, the second sheet 22 and the back sheet 30 extend outward to the longitudinal direction LD in the absorbent core 4.

The sealed portion 50 is formed along the outer perimeter of the absorbent article 1 in portions where this formation is performed in the top sheet portion 2 and the back sheet 30 1-2. Sealed Portion

As shown in Fig. 1, the sealed portion 50 is formed along the entire outer perimeter of the absorbent article 1. A plurality of pressure-bonding parts 51 are formed in the sealed portion 50 by the heat embossing process, and non-pressure-bonding parts 52 are formed between the adjacent pressure-bonding parts 51. The plurality of pressure-bonding parts 51 relatively become concave portions by the heat embossing process in the sealed portion 50, and the non-pressure-bonding parts 52 relatively become convex portions.

The shape of the plurality of pressure-bonding parts 51 is formed in such a way that the length of edges extending along the longitudinal direction LD of the absorbent article 1 is longer than the length of edges extending along the width direction WD of the absorbent article 1. In other words, the shape of the pressure-bonding parts 51 is an elongated shape, which is substantially rectangular, extending long in the longitudinal direction LD of the absorbent article 1. Moreover, the pressure-bonding parts 51 include a shape constituted with a part of the substantially rectangular shape thereof.

The pressure-bonding parts 51 are arranged in the same direction in the sealed portion 50. Specifically, the pressure-bonding parts 51 are arranged in such a way that the edges extending in the longitudinal direction LD in the pressure-bonding parts 51 of the absorbent article 1 are substantially parallel to the longitudinal direction LD of the absorbent article 1. Moreover, the plurality of pressure-bonding parts 51 are arranged in a state where they are separated from each other. Specifically, they are arranged in a state where they are separated from each other in the width direction WD and the longitudinal direction LD of the absorbent article 1.

Moreover, the pressure-bonding parts 51 adjacent to each other in the width direction WD have regions overlapping with the pressure-bonding parts 51 adjacent to each other in the width direction WD. Here, predetermined pressure-bonding parts 51 among the plurality of pressure-bonding parts 51 are assumed be first pressure-bonding parts 53, and pressure-bonding parts adjacent to the first pressure-bonding parts 53 in the width direction WD are assumed to be second pressure-bonding parts 54.

Among the plurality of first pressure-bonding parts 53, the other first pressure-bonding parts 53 adjacent to the predetermined first pressure-bonding parts 53 in the longitudinal direction LD are arranged in series with the first predetermined pressure-bonding parts 53 in the longitudinal direction LD. In other words, edges extending along the longitudinal direction LD in the predetermined first pressure-bonding parts 53 and edges extending along the longitudinal direction LD in the other first pressure-bonding parts 53 are arranged on the straight lines that are substantially parallel to the longitudinal direction LD.

Moreover, the first pressure-bonding parts 53 and the second pressure-bonding parts 54 are arranged to be adjacent to each other in the width direction WD. Specifically, the second pressure-bonding part 54 is arranged to intersect an extension line of an edge which is a straight line extending in the width direction WD from one side in the longitudinal direction LD of first pressure-bonding part 53 of the absorbent article 1. Moreover, the second pressure-bonding part 54 intersecting the extension line from the edge of the one end is not arranged on the extension line from the edge which is a straight line extending along the width direction WD from the other end in the longitudinal direction LD of the first pressure-bonding part 53. In other words, the second pressure-bonding part 54 is arranged to intersect only the extension line of the edge from one end in the longitudinal direction LD of the first pressure-bonding part 53.

As for the positional relationship between the first pressure-bonding part 53 and the second pressure-bonding part 54, it is preferable that the edge extending along the width direction WD from one end of the first pressure-bonding part 53 be arranged in a substantially central position of the length of the edge extending in the longitudinal direction LD of the absorbent article 1 in the second pressure-bonding part 54.

When the absorbent article 1 is observed from the width direction WD, the first pressure-bonding parts 53 and the second pressure-bonding parts 54 have the overlapping regions. Moreover, when the absorbent article 1 is observed from the width direction WD, the non-pressure-bonding parts 52 are not like a continuous straight line extending in the width direction WD in the sealed portion 50.

In the first embodiment, the first pressure-bonding parts 53 and the second pressure-bonding parts 54 have the same shape and the same size. The distance between the first pressure-bonding part 53 and the second pressure-bonding part 54 in the longitudinal direction LD is preferably between 1.0 to 15 mm inclusive, and more preferably between 1.5 and 10 mm inclusive. In the present embodiment, the length of an edge extending in the longitudinal direction LD is 5.6 mm. In cases where the length between the first pressure-bonding part 53 and the second pressure-bonding part 54 in the longitudinal direction LD of the absorbent article 1 is smaller than 1.5 mm, regions occupied by the non-pressure-bonding parts 52 are increased in the width direction WD of the absorbent article 1, thereby it is apprehended that the liquids tend to transfer in the width direction WD. In cases where the length between the first pressure-bonding part 53 and the second pressure-bonding part 54 in the longitudinal direction LD of the absorbent article 1 is greater than 10 mm, the flexibility of the absorbent article 1 in the longitudinal direction LD is decreased, thereby it is apprehended that the absorbent article 1 becomes hard to conform to the shape of the wearer's body.

Moreover, the distance between the first pressure-bonding part 53 and the second pressure-bonding part 54 in the width direction WD is preferably between 0.3 to 5.0 mm inclusive, and more preferably between 0.5 and 2.5 mm inclusive. In cases there the length between the first pressure-bonding part 53 and the second pressure-bonding part 54 in the width direction WD of the absorbent article 1 is smaller than 0.3 mm, it is apprehended that rupture of the top sheet occurs due to the narrowing of the width of the pressure-bonding parts 51. In cases where the length between the first pressure-bonding part 53 and the second pressure-bonding part 54 in the width direction WD of the absorbent article 1 is greater than 5.0 mm, it is apprehended that the rigidity in the pressure-bonding parts 51 is increased, and the flexibility is deteriorated. In the present embodiment, the length of the edge extending in the width direction WD is 1.1 mm.

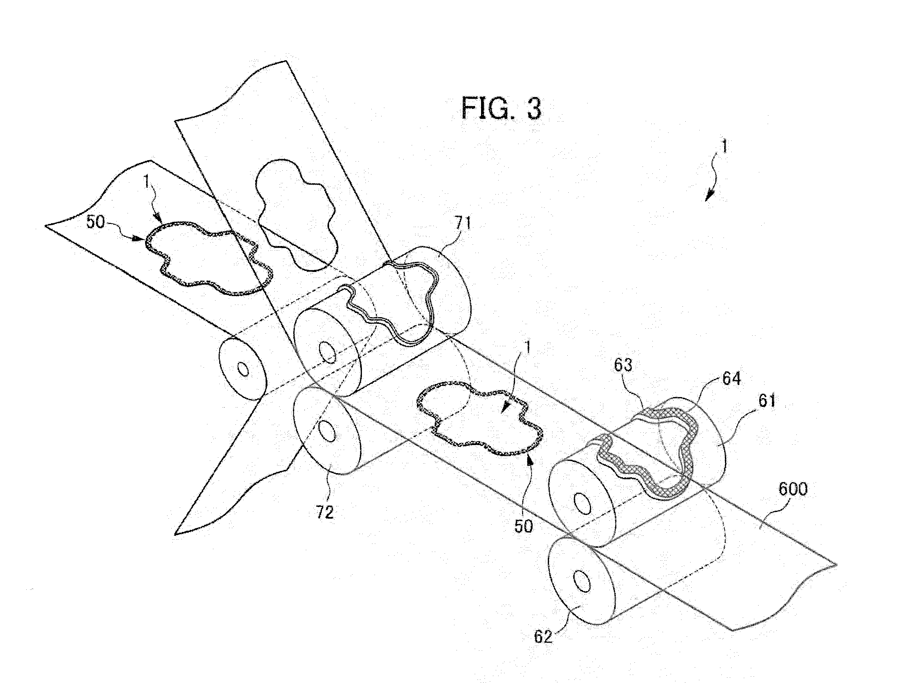

Moreover, the distance between the first pressure-bonding part 53 and the second pressure-bonding part 54 is preferably between 0.9 to 20 mm inclusive. In cases where the distance between the first pressure-bonding part 53 and the second pressure-bonding part 54 is smaller than 0.9 mm, the regions of the non-pressure-bonding parts 52 formed between the first pressure-bonding parts 53 and the second pressure-bonding parts 54 are decreased, thereby it is apprehended that the flexibility is deteriorated. Moreover, in cases where the distance between the first pressure-bonding part 53 and the second pressure-bonding part 54 is greater than 20 mm, the pressure from emboss rolls 61 and 62 (referring to Fig.3) to be described later used for the embossing process becomes uneven when forming the pressure-bonding parts 51 by the embossing process in the manufacturing process of the absorbent article 1, thereby it is apprehended that the durability is deteriorated.

### 1-3. Method of Manufacturing Absorbent Article

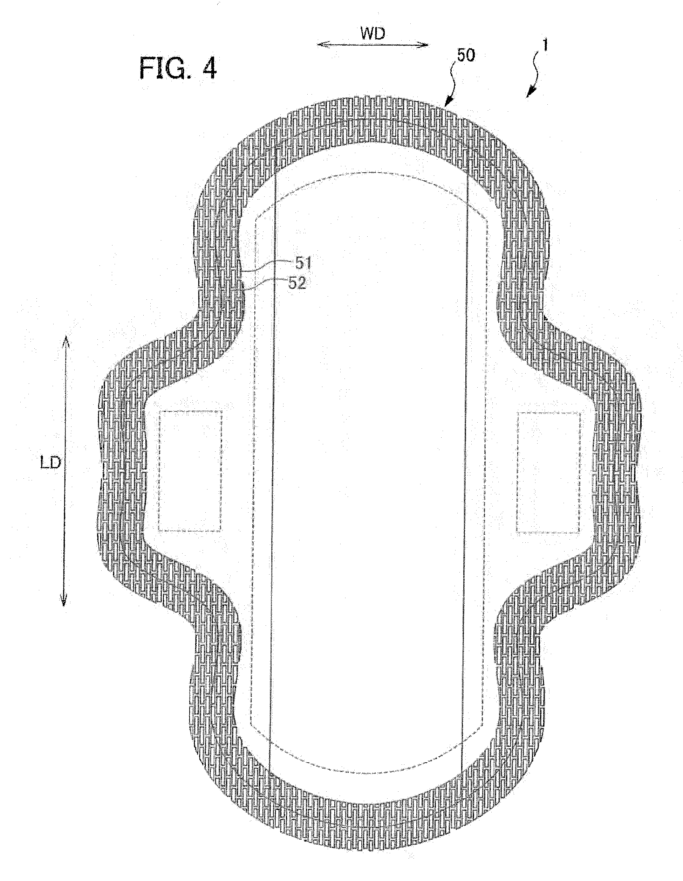

A method of manufacturing the absorbent article 1 is explained with reference to Fig. 3 and Fig. 4. At first, members respectively for a back sheet 30, an absorbent core 4, a second seat 22, a top sheet 20, and side seats 21 are superimposed on a production line in this order in predetermined locations, and each component is joined by a hot-melt adhesive and/or the embossing process, thereby forming an original absorbent article 600.

Next, sealed portion 50 of the absorbent article 1 is formed in the original absorbent article 600. The embossing process is performed in the region to form the sealed portion 50. This embossing process is performed by the emboss rolls 61 and 62. As shown in Fig. 3, a convex portion 64 of the shape corresponding to the shape of the sealed portion 50 is provided to the peripheral face of the emboss roll 61, and a plurality of protrusions 63 corresponding to the shape and pattern of the pressure-bonding parts 51 are formed on the surface of the convex portion 64.

When the original absorbent article 600 passes through between the emboss rolls 61 and 62, a convex portion 64 and the protrusions 63 are pressed against the original absorbent article 600, thereby forming the sealed portion 50 (pressure-bonding parts 51). As shown in Fig. 4, the sealed portion 50 is formed to be wider than the width of the sealed portion 50 in the case where the separation has been performed for the absorbent article 1 in the later process. The reason is because cutter rolls 71 and 72 to be described later cut out the region, in which the sealed portion 50 has been formed, in order to mold the shape of the absorbent article 1.

As shown in Fig. 3, the original absorbent article 600, in which the sealed portion 50 has been formed, passes through between the cutter rolls 71 and 72. The cutter rolls 71 and 72 mold the original absorbent article 600, which passes through between the emboss rolls 61 and 62, into a shape of the absorbent article 1. Specifically, a cutter in the shape of the absorbent article 1 is provided on any one of the cutter rolls 71 and 72, and the cutter cuts out the original absorbent article 600 into the shape of the absorbent article 1, and separates the absorbent article 1.

In the first embodiment, the pressure-bonding parts 51 are formed in a discontinuous manner with predetermined intervals in the sealed portion 50. This makes it possible to reduce the rigidity as compared to the case where the pressure-bonding parts 51 are formed in a planar or linear shape, as a result of which the uncomfortability for the wearer is not caused, and it is possible to maintain the feeling and the soft touch. Moreover, even in cases where the sealed portion 50 of the absorbent article 1 is rubbed by the motion of the wearer, it is possible to obtain a comfortable feeling without uncomfortability while in use.

According to the first embodiment, the pressure-bonding parts 51 are formed in such a way that the edges extending in the longitudinal direction LD of the absorbent article 1 is longer than the edges extending along the width direction WD. This increases the folding-starting points formed by the non-pressure-bonding parts 52 in terms of the width direction WD of the absorbent article 1, thereby making it possible for the absorbent article 1 to deform along the curve of the wearer's body in terms of the width direction WD.

According to the first embodiment, the pressure-bonding parts 51 are formed to extend long in the longitudinal direction LD of the absorbent article 1. This makes it possible to effectively prevent the formation of regions in which the pressure-bonding parts 51 are not provided in the width direction WD, and to prevent the leakage of liquids through the non-pressure-bonding parts 52 that are convex portions.

In the first embodiment, the sealed portion 50 is formed to surround the entire outer perimeter of the absorbent article 1, but it is not limited thereto, and the sealed portion 50 may be formed only in the part. For example, the sealed portion 50 may be formed only in the vicinity of the front edge 8 and the rear edge 9, and may formed partly in each edge of the outer perimeter of the absorbent article 1. Moreover, the sealed portion 50 may be formed in a discontinuous manner with predetermined intervals.

In the first embodiment, the second seat 2 is in a state where one sheet is folded in two, and the length thereof in the longitudinal direction LD is substantially the same as that of the top sheet 20, but it is not limited thereto. The length of the second seat 22 in the longitudinal direction LD is may be shorter than the length of the top sheet 20 in the longitudinal direction LD. In cases where the second seat 22 is smaller, the top sheet 20 and the back sheet 30 are joined each other at the sealed portion 50 in the vicinity of the front edge 8 and the rear edge 9. Moreover, the width of the second seat 22 in the width direction WD may be smaller or larger than that of the top sheet 20. Moreover, the second seat 22 may be used in a state of a single layer instead of a state where it is folded in two, and may be in a state where the second seat 22 and the top sheet 20 are integrated.

In the first embodiment, the pressure-bonding parts 51 are linearly formed, but it is not limited thereto. For example, the pressure-bonding parts 51 may be formed by a curve, or partly by a curve or a straight line.

### 2. Other Embodiments

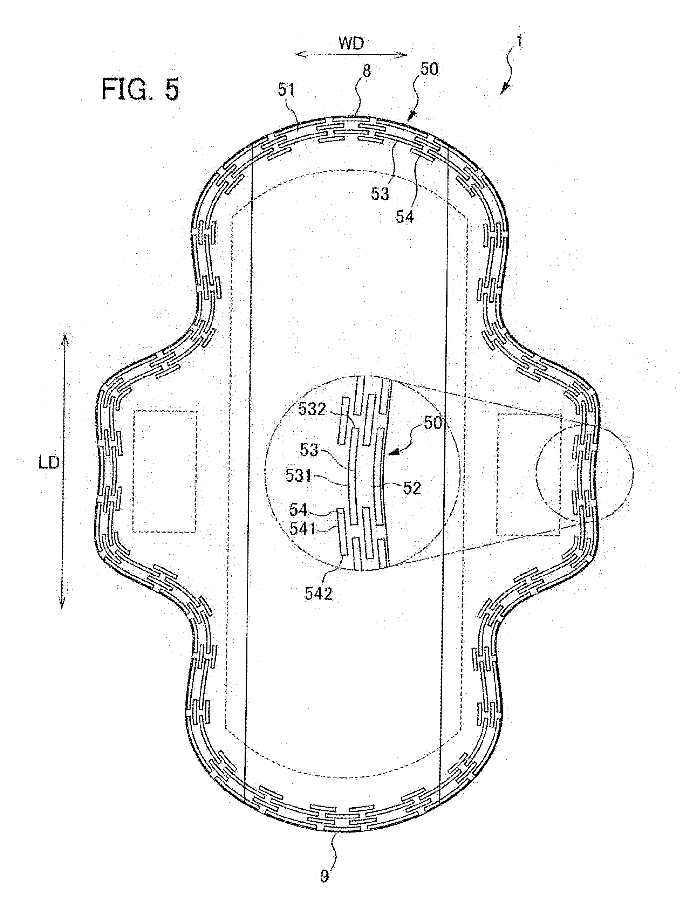

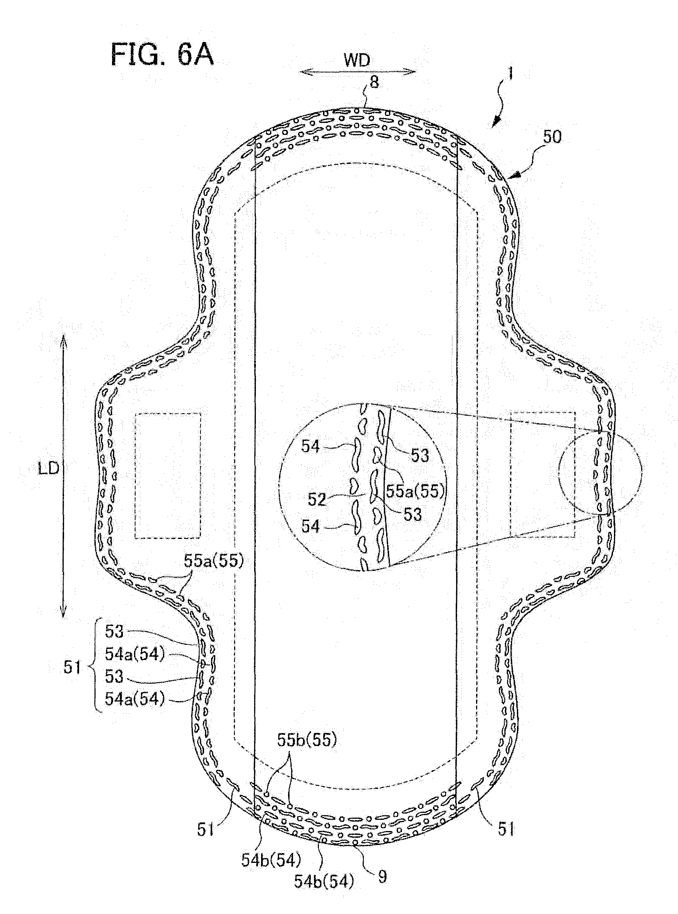

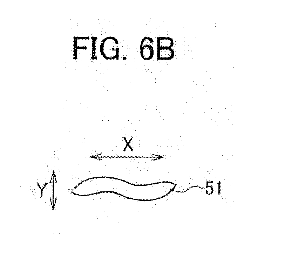

Other embodiments are explained below with reference to Fig. 5 and Fig. 6. The second and third embodiments are different from the first embodiment in the shape or arrangement of the pressure-bonding parts 51 in the sealed portion 50. It should be noted that, parts that are different from the first embodiment are mainly explained below. Moreover, the parts similar to those of the first embodiment are designated by the same numerals, and the description in the first embodiment applies to the parts without a particular description.

### 2-1. Second Embodiment

As shown in Fig. 5, the absorbent article 1 in the second embodiment is different in the shape and arrangement pattern of the pressure-bonding parts 51 in the sealed portion 50 formed along the outer perimeter of the absorbent article 1.

The first pressure-bonding parts 53 and the second pressure-bonding parts 54, in the case of being formed along the longitudinal direction LD of the absorbent article 1, are formed in such a way that edges 531 and 541 extending in the longitudinal direction LD are longer than edges 532 and 542 extending in the width direction WD. Moreover, the edges 532 and 542 are formed substantially in straight line, but the edge 531 and 541 are formed in curve along the outer perimeter of the absorbent article 1. In other words, the first pressure-bonding parts 53 and the second pressure-bonding parts 54 are formed in such a way that, among edges forming the first pressure-bonding parts 53 and the second pressure-bonding parts 54, the longer edges are along the outer perimeter of the absorbent article 1.

In the case of being formed along the longitudinal direction LD of the absorbent article 1, the first pressure-bonding parts 53 and the second pressure-bonding parts 54 are formed in such a way that the length of the first pressure-bonding parts 53 in the longitudinal direction LD is longer than the length of the second pressure-bonding parts 54 in the longitudinal direction LD. Specifically, the length of the edges 531 in the first pressure-bonding parts 53 are longer than the length of the edges 541 in the second pressure-bonding parts 54. Moreover, the length of the direction along the outer perimeter of the absorbent article 1 in the second pressure-bonding parts 54 is formed to be shorter than the length along the outer perimeter in the first pressure-bonding parts 53.

Four lines of the pressure-bonding parts 51 are formed in the sealed portion 50. Specifically, the first line is formed in the outermost perimeter of the sealed portion 50, in which the first pressure-bonding parts 53 and 53 are adjacent to each other along the outer perimeter; the second line is formed inward thereof, in which the second pressure-bonding parts 54 and 54 are adjacent to each other along the outer perimeter; the third line is formed inward thereof, in which the first pressure-bonding parts 53 and 53 are adjacent to each other along the outer perimeter; and the fourth line is formed further inward thereof, in which the second pressure-bonding parts 54 and 54 are adjacent to each other along the outer perimeter.

In this way, the first pressure-bonding parts 53 and the second pressure-bonding parts 54 are arranged alternatingly along the substantially perpendicular direction to the outer perimeter of the absorbent article 1. The second pressure-bonding part 54 has a region overlapping with one end of the edge 531 in the first pressure-bonding part 53 along the direction substantially perpendicular to the outer perimeter of the absorbent article 1. The first pressure-bonding part 53 has a region in the other end of edge 531, in addition to the region overlapping at the one end as described above, which overlaps with the other second pressure-bonding part 54 adjacent in the direction along the outer perimeter of the absorbent article 1, the overlapping being along the direction substantially perpendicular to the outer perimeter of the absorbent article 1.

In the second embodiment, the length of the first pressure-bonding parts 53 along the outer perimeter of the absorbent article 1 is different depending upon the position in the sealed portion 50. Specifically, in terms of the first pressure-bonding parts 53 formed in regions in the vicinity of the apexes in the front edge 8 and the rear edge 9, and in regions in the vicinity of the wings 5L and 5R, the length of the first pressure-bonding parts 53 formed in the other regions in the sealed portion 50 along the outer perimeter of the absorbent article 1 is shorter.

In the sealed portion 50 formed on both sides along the longitudinal direction LD of the absorbent article 1, it is preferable that the distance between the second pressure-bonding part 54 and the other second pressure-bonding part 54 adjacent in the direction of the edge 541 be between 0.7 and 30 mm inclusive. In cases where the distance of the adjoining is smaller than 0.7 mm, the area of the non-pressure-bonding parts 52 in the region is reduced, thereby the flexibility is reduced. Moreover, in cases where the distance of the adjoining is greater than 30 mm, the strength of the entire sealed portion 50 is reduced, thereby the possibility of rupture arises.

Moreover, according to the second embodiment, it is possible to widen the area of the non-pressure-bonding parts 52, thereby making it possible to maintain the flexibility of the absorbent article 1 and to reduce the uncomfortability while in use.

### 2-2. Third Embodiment

As shown in Fig. 6 (A) and Fig. 6 (B), the absorbent article 1 in the third embodiment is different in the shape and arrangement pattern of the pressure-bonding parts 51 in the sealed portion 50 formed along the outer perimeter of the absorbent article 1. In the third embodiment, the pressure-bonding parts 51 include the first pressure-bonding parts 53 as well as the second pressure-bonding parts 54 formed substantially parallel to the first pressure-bonding parts 53. As shown in Fig. 6 (A) and Fig. 6 (B), the first pressure-bonding parts 53 are formed in such a way that the first direction X is substantially parallel to the direction along the outer perimeter of the absorbent article 1, respectively at predetermined intervals for substantially entire circumference of the absorbent article 1. Moreover, the second pressure-bonding parts 54 are formed inward the width direction WD of the first pressure-bonding parts 53, at a predetermined distance from the first pressure-bonding parts 53, in such a way that the first direction X is substantially parallel to the direction along the outer perimeter of the absorbent article 1, respectively at predetermined intervals for substantially entire circumference of the absorbent article 1. That is to say, a broken-line shape formed by the plurality of the first pressure-bonding parts 53, and a broken-line shape formed by the plurality of the second pressure-bonding parts 54 are formed in two lines for substantially entire circumference of the absorbent article 1.

The non-pressure-bonding parts 52 formed between the first pressure-bonding parts 53 and the second pressure-bonding parts 54 are substantially parallel to the direction along the outer perimeter of the absorbent article 1, and are formed in succession in the direction along the outer perimeter. Moreover, the second pressure-bonding part 54 is arranged in such a way that the second pressure-bonding part 54 intersects a straight line extending in the second direction Y from one end of the first pressure-bonding part 53 in the first direction X, but do not intersect a straight line extending in the second direction Y from the other end of the first pressure-bonding part 53 in the first direction X. That is to say, the first pressure-bonding parts 53 and the second pressure-bonding parts 54 are arranged in a hound's tooth check.

In the third embodiment, all of the first pressure-bonding parts 53 have the same shape. Moreover, the second pressure-bonding parts 54a, which are formed on both sides of the longitudinal direction LD of the absorbent article 1, have the same shape as the first pressure-bonding parts 53.

As shown in Fig. 6 (B), the shape of the first pressure-bonding parts 53 and the second pressure-bonding parts 54a, which are formed on both sides of the longitudinal direction LD of the absorbent article 1, is formed in such a way that the length in the first direction X is greater than the length in the second direction Y. Moreover, it is possible to form, with a curve, each or part of edges that form the second pressure-bonding parts 54a formed on both sides of the longitudinal direction LD of the first pressure-bonding parts 53 and the absorbent article 1. In the third embodiment, the outer circumference of the second pressure-bonding parts 54a, which are formed on both sides of the longitudinal direction LD of the first pressure-bonding parts 53 and the absorbent article 1, is formed with a curve. The first pressure-bonding parts 53 has a shape which is meandering as a whole from one end to the other end of the first direction X, with the both ends being apexes of the shape. The second pressure-bonding parts 54a, which are formed on both sides of the longitudinal direction LD of the first pressure-bonding parts 53 and the absorbent article 1, have a shape in which both edges extending in the first direction X meander substantially in parallel to each other, the edges intersecting at both ends of the first direction X to form apexes.

In the sealed portion 50 in the vicinity of the front edge 8 and the rear edge 9 of the absorbent article 1, the shape of the second pressure-bonding parts 54b is different from the shape of the second pressure-bonding parts 54a formed on both sides of the longitudinal direction LD of the absorbent article 1. The shape of the second pressure-bonding parts 54b is formed with a curve, in which edges extending in the first direction X bulge outward at a substantial center of the first direction X, with both ends of the first direction X being apexes.

Moreover, in the sealed portion 50 in the vicinity of the front edge 8 and the rear edge 9 of the absorbent article 1, the second pressure-bonding parts 54 are formed inward the absorbent article 1, and the first pressure-bonding parts 53 are formed respectively at predetermined intervals in such a way that the first direction X of the first pressure-bonding parts 53 is substantially in parallel to the direction along the outer perimeter of the absorbent article 1. In addition, further inward thereof, the second pressure-bonding parts 54b are formed respectively at predetermined intervals in such a way that the first direction X is substantially in parallel to a direction along the outer perimeter of the absorbent article 1. That is to say, in the sealed portion 50 in the vicinity of the front edge 8 and the rear edge 9 of the absorbent article 1, a broken-line shape formed of the first pressure-bonding parts 53 and a broken-line shape formed of the second pressure-bonding parts 54b are alternately formed in four lines in total.

In the third embodiment, as described above, the sealed portion 50 includes third pressure-bonding parts 55 of which the shape is different from that of the plurality of pressure-bonding parts 51 (the first pressure-bonding parts 53 and the second pressure-bonding parts 54). The third pressure-bonding part 55 is provided between the predetermined first pressure-bonding part 53 and the other first pressure-bonding parts 53 adjacent to this first pressure-bonding part 53 in the direction of the outer perimeter of the absorbent article 1. The third pressure-bonding part 55 is formed between the two second pressure-bonding parts 54 and 54 that are adjacent to each other along the direction of the outer perimeter of the absorbent article 1, and between the two first pressure-bonding parts 53 and 53 that are adjacent to each other in the direction of the outer perimeter of the absorbent article 1. In other words, the plurality of pressure-bonding parts 51 and the plurality of third pressure-bonding parts 55 are alternately formed along the direction of the outer perimeter of the absorbent article 1.

The third pressure-bonding parts 55 assist the joining of the top sheet portion 2 and the back sheet 30 by the first pressure-bonding parts 53 and the second pressure-bonding parts 54. In the third embodiment, the third pressure-bonding parts 55 include heart-shaped third pressure-bonding parts 55a that are formed on both sides of the longitudinal direction LD of the absorbent article 1, and substantially circular third pressure-bonding parts 55b that are formed in the vicinity of the front edge 8 and the rear edge 9 of the absorbent article 1.

Thus, in the third embodiment, on both sides of the longitudinal direction LD of the absorbent article 1, a broken-line shape, in which the first pressure-bonding parts 53 and the heart-shaped third pressure-bonding parts 55a are alternately arranged, is formed to the side of the outer perimeter thereof, and a broken-line shape, in which the second pressure-bonding parts 54a and the heart-shaped third pressure-bonding parts 55a are alternately arranged, is formed inside thereof. Moreover, in the sealed portion 50 in the vicinity of the front edge 8 and the rear edge 9 of the absorbent article 1, a broken-line shape, in which the first pressure-bonding parts 53 and the substantially circular third pressure-bonding parts 55b are alternately arranged, and a broken-line shape, in which the second pressure-bonding parts 54b and the substantially circular third pressure-bonding parts 55b are alternately arranged, are alternately formed in four lines from the outer perimeter.

In the boundary between the front edge 8 as well as the rear edge 9 in the absorbent article 1 and the side edges along the longitudinal direction LD of the absorbent article 1, a region is formed in which the number of the pressure-bonding parts 51 is small. In the third embodiment, in the side edges to the longitudinal direction LD in the vicinity of the boundary between the front edge 8 as well as the rear edge 9 and the side edges to the longitudinal direction LD, regions are formed in which the pressure-bonding parts 51 are formed in only one line.

In this way, the shape and the number of the pressure-bonding parts 51 to be formed can be different depending on the position in the outer perimeter of the absorbent article 1. As a result, the pattern as a whole to be formed by the pressure-bonding parts 51 is different depending on the position in the sealed portion 50, thereby making it possible to improve the visibility.

Moreover, the formation is performed in such a way that the number of the pressure-bonding parts 51 to be formed in the sealed portion 50 in the vicinity of the front edge 8 and the rear edge 9 is larger than the number of the pressure-bonding parts 51 to be formed on both sides along the longitudinal direction LD of the absorbent article 1. This makes it possible to vary the rigidities of the sealed portion 50 depending upon the position in the sealed portion 50.

In the third embodiment, the pressure-bonding parts 51 are formed in such a way that the shape along the outer perimeter of the absorbent article 1 and the length along the outer perimeter are different, thereby making it possible to suppress the increase of the rigidity as a whole.

The present invention is not limited to the above described embodiments, but various alterations are possible within a scope of the present invention. For example, the shape of the pressure-bonding parts 51 is not limited to the shapes as described in the above mentioned embodiments, and the shape of the first pressure-bonding parts 53 and the shape of the second pressure-bonding parts 54 may be different over the entire outer perimeter of the absorbent article 1. Moreover, the material of each component constituting the absorbent article 1 is not limited to the above described material, but it is possible to use materials used for conventional absorbent articles as appropriate.

The absorbent article of the present invention may be, in addition to sanitary napkins, urine-absorbing pads, panty liners, disposable diaper and the like.

## Claims

1. An elongated absorbent article comprising:
a top sheet portion having a seat, at least a part of which is liquid-permeable;
a back sheet portion having a liquid-impermeable seat;
a liquid retentive absorbent core which is arranged between the top sheet portion and the back sheet portion;
a joined region formed in outer perimeter of the absorbent article, which joins the back sheet portion and the top sheet portion; and
a plurality of pressure-bonding parts are formed in a separated way in all or part of the joined region, and
wherein each of the plurality of pressure-bonding parts formed in the outer perimeter in a width direction of the absorbent article has a region overlapping with an pressure-bonding part adjacent to each other in the width direction.

2. The absorbent article according to claim 1,
wherein the plurality of pressure-bonding parts has a plurality of first pressure-bonding parts formed along the outer perimeter and a plurality of second pressure-bonding parts formed to be substantially parallel to the first pressure-bonding parts, and
wherein the second pressure-bonding parts are formed to intersect a straight line extending in a width direction from one end of a longitudinal direction of the first pressure-bonding parts, and not to intersect a straight line extending in the width direction from another end of the longitudinal direction.

3. The absorbent article according to claim 2,
wherein, in the longitudinal direction, the length of the first pressure-bonding parts are different from the length of the second pressure-bonding parts.

4. The absorbent article according to any one of claim 2 or 3, further comprising third pressure-bonding parts, each of which is arranged between a predetermined first pressure-bonding part in the plurality of first pressure-bonding parts and the other first pressure-bonding part adjacent to the predetermined first pressure-bonding part, and of which the length is different in the longitudinal direction from the length of the first pressure-bonding parts.

5. The absorbent article according to any one of claims 1 to 4,
wherein all or part of the plurality of pressure-bonding parts has a shape elongated in the longitudinal direction of the absorbent article.

6. The absorbent article according to any one of claims 1 to 5,
wherein the plurality of pressure-bonding parts are formed in the entire area of the outer perimeter, and have regions overlapping with adjacent pressure-bonding parts in the width direction with respect to any position in the longitudinal direction of the absorbent article.
